Europäisches Patentamt

European Patent Office

Office européen des brevets

(11) Veröffentlichungsnummer: **0 208 805**
**A1**

# (12) EUROPÄISCHE PATENTANMELDUNG

(21) Anmeldenummer: 85109079.5

(22) Anmeldetag: 19.07.85

(51) Int. Cl.4: **C12N 5/00** , C12N 1/06 ,
A01N 63/00 , A01N 65/00 ,
A23K 1/16 , A23L 1/30 ,
A61K 7/48 , A61K 35/66 ,
A61K 35/72 , A61K 35/78

(43) Veröffentlichungstag der Anmeldung:
21.01.87 Patentblatt 87/04

(84) Benannte Vertragsstaaten:
AT BE CH DE FR GB IT LI LU NL SE

(71) Anmelder: **Rotter, Ulrich Dipl.Chem.**
**Brunnerstrasse 27**
**D-8000 München 40(DE)**

(72) Erfinder: **Wank, geb. Stendel, Anna**
**An der Apostelkirche 5III**
**1000 Berlin 30(DE)**

(74) Vertreter: **Rotter, Ulrich, Dipl.-Chem. Dr. et al**
**Patentanwälte Dipl.-Ing. Olaf Ruschke**
**Dipl.-Ing. Hans E. Ruschke Dipl.-Ing. Jürgen**
**Rost Dipl.-Chem. Dr. U. Rotter**
**Pienzenauerstrasse 2**
**D-8000 München 80(DE)**

(54) **Verfahren zur Herstellung thallophytischer oder bryophytischer Zytorrhysate, danach hergestellte Chemizytorrhysate und deren Verwendung zur Nährstoffergänzung.**

(57) Zur Herstellung von thallophytischen bzw. bryophytischen Zytorrhysaten wird vorgeschlagen, derartige intakte Zellen chemizytorrhytisch in der Weise zu behandeln, daß sie zunächst bei 0° bis 4o°C mit einem wasserlöslichen Alkali-oder Erdalkalisalz unter Verflüssigung gemischt und dann auf eine Temperatur über 4o°C, jedoch unterhalb der Pasteurisierungstemperatur erwärmt und schließlich abgekühlt werden. Diese Chemizytorrhysatzellmassen eignen sich als Nährstoffergänzung zu Futter und Trinkwasser, als Pflanzenwuchsstoff, als Kosmetikwirkstoff zur Aktivierung des Hautstoffwechsels und als Aphrodisiakum zur Potenzsteigerung.

### Verfahren zur Herstellung thallophytischer oder bryophytischer Zytorrhysate, danach hergestellte Chemizytorrhysate und deren Verwendung zur Nährstoffergänzung

Die Erfindung betrifft die Herstellung von chemizytorrhysierten Zellen von Thallophyten und Bryophyten sowie derartige Chemizytorrhysate, die den Zellstoffwechsel tierischer und pflanzlicher Organismen aktivieren können, und zwar im Sinne von Nahrungs-und Futtermittelzusatzstoffen für Mensch und Tier, im Sinne von Pflanzenwuchsstoffen für Pflanzen verschiedenster Art. Präparate, die die erfindungsgemäß hergestellten Chemizytorrhysate enthalten, sind außergewöhnlich wirksame,die Zellproliferation stimulierende Mittel auf der Basis von CACCC-Faktoren (CACCC steht für "cell metabolism activating chemicytorrhysized cells").

Zellstoffwechsel aktivierende Faktoren sind bekannt. Zum Beispiel fördert ein von Proteinen und anderen höhermolekularen Bestandteilen befreiter Extrakt aus Blut die Zell atmung tierischer und menschlicher Zellen und erhöht die Sauerstoffaufnahme bei isolierten Mitochondrien (Jaeger et al., Arzneimittelforschung 1965, S. 750-4). Zur Verbesserung der Zellatmung und Hirnfunktion des Menschen sind Präparationen aus Kälberblutextrakten mit Heptaminol-Zusatz vorgeschlagen worden. Gibberelline sind bekannte Pflanzenwuchsstoffe, die das Wachstum vieler Pflanzen schon in sehr kleinen Mengen stark beeinflussen können; ihre Dosierung ist jedoch schwierig. Als Futtermittelzusatzstoffe kommen die zellstoffwechsel-aktivierenden Faktoren (Extrakte) nicht in Frage, wie entsprechende Versuche gezeigt haben.

Die Wirkung von Östrogenen, Antibiotica, Arsen-und Kupferverbindungen als Futtermittelzusatz ist den genannten Extrakten deutlich überlegen.

Stimulatoren bzw. Regulatoren zur Ernährung von Tieren und Pflanzen, vor allem domestic animals und Nutzpflanzen, sind immer häufiger in Gebrauch, um die Gesundheit und das Wachstum zu verbessern und günstig zu beeinflussen. Es sind Präparate, die selbst keine Nahrungsmittel bzw. Nährstoffe für Tiere bzw. Pflanzen sind, die eine prophylaktische Wirkung entfallen und den anabolischen Stoffwechsel beeinflussen.

Jedoch sind für alle bisher in diesem Zusammenhang entwickelten Verbindungen und Präparate Nebenwirkungen beobachtet worden, denen heute verstärkt die Aufmerksamkeit durch die Öffentlichkeit zugewandt wird. Dies gilt z.B. für Verbindungen wie Zinkbacitracin (ein Antibiotikum der Peptolid gruppe), für Nitrovin (1,5-Di(5-nitro-2-furfuryl)-1,4-pentadien-3-on-amidinhydrazon.HCl) der Furangruppe, für Arsanilsäure (p-Aminophenylarsinsäure), für Dienoestrol-diacetat (ein Östrogenderivat), für Carbadox (Methyl-3-(2-chinoxalinyl)-methylen)carbazat-N¹,N⁴-dioxid) aus der Chinoxalindioxidgruppe, für Kupfersulfat u.a..

Die ausgesprochenen Pflanzenwuchsstoffe wie Auxine, Heteroauxine, Gibberelline u.a. sind in ihrer Wirkung sehr konzentrationsabhängig und - schwierig zu dosieren, ganz abgesehen von zum Teil hohem Preis und von ihrer Spezifität.

Der Erfindung liegt die Aufgabe zu Grunde, thallophytische und bryophytische Zytorrhysate auf CACCC-Basis herzustellen, die ausschließlich auf natürlichen Materialien (Thallophyta, Bryophyta) basieren, frei von Nebenwirkungen sind, die den bisherigen, oben genannten Futterzusatzstoffen und Pflanzenwuchsstoffen anhaften, und wirksame Nahrung-und Futterzusätze und Pflanzenwuchsstoffe darstellen.

Hierzu schlägt die Erfindung das in Anspruch 1 angegebene Verfahren zur Herstellung von Zytorrhysaten von Thallophyten und Bryophyten vor. Bevorzugte Ausgestaltungen dieses Verfahrens sind Gegenstand der Unteransprüche 2 bis 12 . Das nach diesem Verfahren erhältliche Chemizytorrhysat und dessen Verwendung für die Nährstoffergänzung sind Gegenstand der Ansprüche 13 bis 17.

Überraschenderweise hat sich gezeigt, daß durch Chemizytorrhyse behandelte Thallophyten und Bryophyten eine hervorragende Basis für CACCC-Präparate sind, die als Futtermittelzusätze, Nahrungsmittelzusätze, Pflanzenwuchsstoffe, und sogar als Kosmetika und Aphrodisiaka verwendbar sind. Ihre Anwendung erstreckt sich auf den Tier-und Pflanzenbereich.

Die nach dem erfindungsgemäßen Verfahren erhaltenen Chemizytorrhysate erwiesen sich nach den üblichen toxikologischen Methoden als ungiftig und im Screening-Test als nichttoxisch. Die $LD_{50}$ liegt bei oraler Gabe über 2o g/kg. Hautreizungen, insbesondere bei Augen von Kaninchen, konnten nicht beobachtet werden.

Die Verwendbarkeit als Aphrodisiakum ergibt sich aus folgenden Beobachtungen und Experimenten:

Es ist bekannt, daß Hefe die Sexualität von Mäusemännchen hemmt, wenn normale unbehandelte Hefe in einer Menge von 0,5 % dem Trinkwasser hinzugefügt wird. Diese Hemmung wird aufgehoben, wenn dem Trinkwasser 0,15 % der erfindungsgemäßen zytorrhysierten Hefe (hergestellt wie in Beispiel 3 ) hinzugefügt werden. Die Ergebnisse sind in Tab. I zusammengefaßt. Präparationen mit den Chemizytorrhysaten der Beispiele 1 und 2 zeigten ähnliche Resultate.

Tabelle I: Hemmung der Sexualität von Mäusen durch Hefe und Kompensation durch zytorrhysierte Hefe - je Versuch 50 männl. Tiere und 200 weibl. Tiere (1:4)

| Versuchs-reihe Nr. | Zahl d. männl. Mäuse | Hemmung der Sexualität d. normale Hefe (%)* | nach Aufheben d. Hemmung d. Zugabe von zytorrhysierter Hefe* | |
|---|---|---|---|---|
| | | | Hemmung (%) | Steigerung (%) |
| 1 | 50 (1:4) | 70 | 0 | 20 |
| 2 | 50 (1:4) | 65 | 10 | - |
| 3 | 50 (1:4) | 60 | 0 | 25 |

\* Hemmung bzw. Steigerung gemessen an der Zahl der Jungen pro 50 Würfe, bezogen auf Kontrollen ohne Hefe

Beim erfindungsgemäßen Verfahren werden intakte Thallophytzellen, Bryophytzellen oder beide chemizytorrhytisch behandelt, indem die Zellen bei Temperaturen im Bereich von etwa 0°C bis 40°C, bevorzugt bei 25°C bis 35°C, innig mit einem wasserlöslichen Salz und vorzugsweise einem Salzgemisch aus Salzen der Metalle der 1. und 2. Hauptgruppe des Periodensystems gemischt werden, wobei die Salzmenge so bemessen wird, daß sie zur Verflüssigung der Zellkörpermenge ausreicht. Danach wird das verflüssigte Gemisch, vorzugsweise rasch, auf eine Temperatur erwärmt, die über 40°C liegt, jedoch unterhalb der Pasteurisierungstemperatur der Milch (82°C) liegen sollte. Nach Erwärmen auf eine erhöhte Temperatur, das bevorzugt mindestens 10 min dauert, wird das Gemisch abgekühlt, ggf. mit Konservierungsstoffen versetzt, und ist gebrauchsfertig. Es ist vorteilhaft, die fertige Hefesuspension in festem Kieselgel aufzunehmen und hierdurch in eine günstige versandfähige Form zu bringen.

Die Hefesuspension kann selbstverständlich auch in Materialien aufgenommen werden, die üblicherweise als Viehfutter Verwendung finden, wie zum Beispiel in Maismehl und anderen Futtersorten.

Die Verfestigung der Hefesuspension kann auch durch Entfernen des Wassers im Vakuum erreicht werden.

Sehr vorteilhaft und deshalb bevorzugt ist die Entfernung der Hauptmenge an Feuchtigkeit der Hefesuspension, indem man Sprühtrocknungsverfahren anwendet, wobei darauf zu achten ist, daß die thermische Beanspruchung des Produktes so kurzzeitig und so gering wie möglich bleibt, was zum Beispiel mit einem Umsatz von 2.000 kg/Std. gewährleistet ist.

Zur Einleitung der chemizytorrhytischen Reaktionen werden die Zellen der nachfolgend im einzelnen genannten Materialien und anderer zu den Thallophyten und Bryophyten gehörenden pflanzlichen Organismen, einschließlich der Bakterien, mit Salzen und bevorzugt mit Salzgemischen aus Salzen von Metallen der 1. und der 2. Hauptgruppe des Periodensystems behandelt. Geeignete Metall salze sind die des Natriums, Kalium und Lithiums sowie Salze des Calciums, Magnesiums und Berylliums. Im Zuge der Zytorrhyse verringert sich der Wassergehalt innerhalb der Zellen, ohne daß in der Bruttobilanz Wasser aus dem Gesamtverband verloren geht. Die Zuführung von wäßrigen Systemen von außen, zum Beispiel die Anwendung von wäßrigen verdünnten Salzlösungen, ist demgegenüber unerwünscht und sogar schädlich, da die Chemizytorrhyse hierdurch beeinträchtigt wird. Es hat sich gezeigt, daß Chemizytorrhyse nur dann möglich ist, wenn Wasser aus der Zelle entfernt werden kann.

Als Osmocitica kommen die meisten Salze in Betracht, die eine nicht zu geringe Wasserlöslichkeit besitzen, bevorzugt werden die Natrium-und Kaliumsalze sowie die Calcium-und Magnesiumsalze. Bevorzugte Anionen, insbesondere in Verbindung mit den vorgenannten bevorzugten Metallionen, sind Sulfate, Nitrate, Chloride, Acetate, Citrate, Lactate, Malate, Succinate, Carbonate, Bicarbonate und Phosphate.

Die Mengenverhältnisse von Zellmaterial zu Salzgemisch werden allgemein so eingestellt, daß eine Mindestmenge an Salzgemisch, die zur Verflüssigung ausreicht, eingesetzt wird. Vorzugsweise liegt diese Menge im Bereich von 1,5 -2,5 % des Zellkörpermaterials; 2,0 % ± 0,2 % werden speziell bevorzugt. In den meisten Fällen schadet ein Überschuß an Salz jedoch nicht.

Es hat sich als nützlich erwiesen, den Salzgemischen einen Zusatz an Kohlenhydraten und/oder Alkoholen hinzuzufügen. Geeignete Kohlenhydrate sind Glukose, Saccharose, Maltose, Lactose, Fruktose, Mannose, Sorbose, Fucose, L-Ascorbinsäure, Glukosamin, Glukonsäure, Glucuronsäure, Cyclodextrine, Glykogen, lösliche Stärke. Geeignete Alkohole sind: Glycerin, Glykol, Mannit, Sorbit, Ethanol, Pentaerythritol. Eine Kombination aus Salzgemisch und mehrwertigen Alkoholen ist günstig und wird bevorzugt.

Der erste Teil der Chemizytorrhyse wird bei Temperaturen von etwa 0°C bis 40°C durchgeführt. Nach Einwirkung der Osmocitica wird im Anschluß an die Verflüssigung des Zellmaterials auf Temperaturen erwärmt, die vorzugsweise zwischen 40°C und 70°C liegen, meistens und bevorzugt aber nicht über 63°C hinausreichen. Die Zeitdauer richtet sich im einzelnen nach der eingesetzten Menge, sie sollte jedoch vorzugsweise 10 min nicht unterschreiten.

Vorzugsweise wird die verflüssigte Präparation rasch auf die Endtemperatur erwärmt, was vorteilhaft durch Vorerwärmen des Rührgefäßes z.B. auf 70°C erreicht werden kann.

Eine andere, wenn gleich weniger bevorzugte Arbeitsweise besteht darin, das Zellmaterial zunächst zu erwärmen (unter den oben angegebenen Bedingungen) und erst dann das Salzgemisch zuzusetzen.

Es hat sich als besonders günstig herausgestellt, wenn der Anteil des Salzes eines Metalls der 2. Hauptgruppe etwa 10 % der Menge des Salzes des Metalls bzw. der Metalle der 1. Hauptgruppe ausmacht.

Bei Mitverwendung von Kohlenhydraten und/oder Alkoholen beläuft sich deren Menge auf bevorzugt, 0,5 bis 1,5 Gew.-% der Zellkörpermasse (vor Entwässerung).

Der hier benutzte Begriff der Zytorrhyse ist aus der Literatur bekannt (vgl. Zeitschrift "Die Brauwelt", Heft 12, 1946, S. 183 -185). In Verbindung mit der vorliegenden Erfindung steht der Begriff Zytorrhyse für Wasserentzug aus Zellen durch Osmotica und Wärmeeinwirkung. Kressin definiert Zytorrhyse als Zellkontraktion durch Wasserentzug bzw. Wasserabgabe aus Zellen unter dem Einfluß von Hitze, Kälte oder Osmotica. Voraussetzung für Zytorrhyse ist eine Zellhaut, die im Gegensatz zu der fast kastenartigen Festigkeit der Zellwand einer im Gewebsverband stehenden Zelle einer höheren Pflanze eine hohe Dehnbarkeit besitzt. Ferner sind wichtige Faktoren die Permeabilität für Wasser, nicht aber für Plasmolytika und die Tatsache, daß das Protoplasma durch Wasserabgabe zu einem Gel mit den Eigenschaften eines hochquellbaren Kolloids wird.

Im Zuge der Erfindung wurde festgestellt, daß sich bei der erfindungsgemäßen Zytorrhyse folgende Vorgänge vollziehen:

1) Zytorrhyse im klassischen Sinne (Kressin), ohne bedeutsame Plasmolyse.

2) Partieller Wasserentzug aus den Zellen geeigneter Thallophyta und Bryophyta durch Osmotica und Hitze (Chemizytorrhyse).

3) Eliminierung von Hemmstoffen (Inhibitoren) des Wachstums, der Proteinsynthese und der Sexualität (Chemizytorrhyse).

4) Bildung von Stoffwechselaktivatoren, sowohl Erhöhung der Konzentrationen vorhandener als auch Bildung neuer Aktivatoren (Chemizytorrhyse).

5) Schutz der Zellkomponenten durch Membranverstärkung, so daß die chemizytorrhysierten Zellen eine Depotwirkung entfalten können bzw. an Orte gelangen können, die von den nicht-zytorrhysierten Zellen nicht erreicht werden (Zytorrhyse).

Diese Wirkungen der Chemizytorrhyse sind besonders ausgeprägt bei Präparaten, die mit Salzgemischen in den bevorzugt angewendeten Mengen hergestellt sind, welche Salzgemische sowohl Salze der 1. Hauptgruppe als auch der 2. Hauptgruppe, bevorzugt Salze des Natriums und Magnesiums enthalten. Der Zusatz der Kohlenhydrate und/oder Alkohole, bevorzugt Saccharose und Glucose, verstärkt diese chemizytorrhytische Wirkung.

Erfindungsgemäß werden Zellen von Thallophyten und Bryophyten chemizytorrhytisch behandelt. Thallophyten umfassen die bekannten und frei zugänglichen Algae und Fungi, Bryophyten die bekannten und frei zugänglichen Klassen der Hepaticae (liverworts), Anthrocerotae (horned liverworts) und Musci mosses.

Zu den Fungi gehören die Klassen der Phycomycetes algal fungi (Mucor, Pythium, Phytophora) und Ascomycetes sac fungi (Saccharomyces, Aspergillus, Neurospora) und der Basidiomycetes club fungi (Puccinia, Amanita, Polyphorus) sowie Fungi imperfecti.

Zu der Algae gehören die folgenden Klassen: Chlorophytagreen algae, Chrysophyta -yellow-green algae, golden brown algae, Pyrrhophyta, Phaeophyta -brown algae, Cyanophtya -blue-green algae, Rhodophyta -red algae, Schizomycota -bacteria (Streptococcus, Bacillus, Spirillum) und Myxomocyta -slime mollds.

Aus der Klasse der Ascomycetes kommen von der Gattung der hefeartigen Pilze bevorzugt die folgenden Arten in Frage: Schizosaccharomyces (Hirsebier in Afrika, auch in Melasse auftretend); Endomyces lactis (Milchschimmel, der als Nützling bei der Käsereifung auftritt); Saccharomyces (Hefen im engeren Sinne), und zwar Saccharomyces cerevisiae (Backhefen, Brennereihefen und untergärige Bierhefen), Saccharomyces cerevisiae var. ellipsoideus (Weinhefen), S. carlsbergensis -(untergärige Bierhefen), S. fragilis JÖRGENSEN; S. lactis DOMBROWSKI, S. rosei GUILIERMONT, S. biosoprus NAGARISHI;Hansenula-Hefen wie H. anomala HANSEN-SYDOW; Trichosporon cutaneum OTA (als Begleiter der Back-und Futterhefe); Candida tropicalis und C. utilis HENNEBERG L. u. KR -(Torulopsis utilis), die technische Futter-und Nährhefen darstellen, Candida Krusei (Rahmhefe) BERKHOUT; Kloeckera JANKE (Weinmoste); Sporobolomycetes (Getreide, Süß-und Seewasser, Heu und Stroh); und Pichia polymorpha KLÖCKER (Pichia HANSEN).

Bevorzugte Chemizytorrhysate werden mit den Hefen im engeren Sinne (Saccharomyces) und technischen Futter-und Nährhefen (C. tropicalis, Torulopsis utilis) erhalten.

Weitere bevorzugte Ascomycetes sind Aspergillus niger und Neurospora crassa.

Aus den Klassen der Algen werden besonders die Arten Chlorella, Nitschia, Scenedesmus, Bacillus bifidus und Streptococcus bevorzugt.

Von den Moosen werden Blattmoose (z.B. Mnium und Catharinea) bevorzugt.

Die erfindungsgemäß hergestellten Chemizytorrhysate werden als Suspensionen mit ca. 30 bis 35 % Feststoffgehalt nach der chemizytorrhytischen Osmotica-und Wärmebehandlung erhalten. Sie können problemlos mit Wasser oder wäßrigen Lösungen verdünnt werden. Bevorzugt werden sie als Nährstoffergänzung oder Wuchsstoffe in Mengen von etwa 0,1% bis 2 % dem Normalfutter bzw. Trinkwasser bzw. beiden zugesetzt. Derartige Fut-terzusätze zeigen teilweise verblüffende Ergeb-nisse. So waren Guppifische, die mit einem Futter gezogen wurden, das ein Chemizytorrhysat der Erfindung auf der Basis von Aspergillus niger und Torula utilis (1:1) und 2 bis 4 Gew.-% eines Salzgemisches (Gemisch aus Citraten, Lactaten und Chloriden des Natriums und Magnesiums mit einem Na:Mg-Gewichtsverhältnis von 10:1) enthielt, bereits nach 4 Wochen um 95 % größer als normal gefütterte Kontrollfische.

In vielen Fällen ist es notwendig, das Chemizytorrhysat nicht als Suspension, sondern in fester Form zur Anwendung zu bringen, da viele Futtermittel in fester Form verabreicht werden.

Wie bereits erwähnt, läßt sich durch eine Sprühtrocknung rasch ein festes Produkt erhalten, wenn möglichst milde Bedingungen eingehalten werden. Aber auch durch Gefriertrocknung oder durch Wasserentzug im Vakuum bei niedriger Temperatur oder durch Aufsaugenlassen der Suspension von einem geeignete-Medium, das dann weiterverdünnt werden kann, gelangt man zu einem festen Chemizytorrhysat.

Die Wirkung der die erfindungsgemäßen Chemizytorrhysate enthaltenden Präparate auf CACCC-Basis kann direkt und/oder indirekt sein. Eine indirekte Wirkung liegt beispielsweise vor, wenn ein regulierender Einfluß auf schädliche Mikroorganismen, z.B. der Darmflora von Warm-und Kaltblütern, oder von Blattläusen eintritt, so daß das biologische Gleichgewicht wieder hergestellt werden und damit eine bessere Ausnutzung der Nährstoffe erfolgen kann (Ausbalancieren der Darmflora, ohne sie zu zerstören).

Solche Ungleichgewichte der Darmflora von Tieren und Menschen können durch die verschiedensten Umstände verursacht sein, z.B. durch Transport, widrige Jahreszeiten, ungenügendes Wachstum, Futteränderung und Änderung der Um-gebung. Erfindungsgemäß hergestellte Chemizy-torrhysate enthaltende Präparate eignen sich aufgrund dieses überraschenden Effektes auf die Darmflora (medikamentöser Effekt) in besonderem Maße auch zur Rekonvaleszenz.

Die Wirksamkeit der erfindungsgemäß zytorrhysierten Zellen geeigneter Objekte zeigt sich beispielsweise bei dem im folgenden beschriebenen Kaninchen-Versuch, bei dem das nach Beispiel 3 hergestellte Präparat eingesetzt worden ist.

Unter Verwendung der Wachstumsparameter: Körpergewicht, Futterverbrauch, Wasseraufnahme und Organgewicht wurde das Wachstumsverhalten von je 15 Kaninchen männl. und weibl. Geschlechts (Albinokaninchen, weiße Neuseeländer) mit Gewichten von 1,4 kg bis 2,0 kg beobachtet. Die Laborstandarddiät: Höing und Wasser ad libitum. Das Test-Präparat wurde dem Trinkwasser in einer Menge von 0,1 % hinzugefügt. Die Kaninchen wurden bei Raumtemperatur von 20°C und rel. Feuchtigkeit von 50 -60 % gehalten.

Klinisches Bild: Die Tiere zeigten nach der 6wöchigen Behandlungsdauer keine Abweichungen vom Normalverhalten (ersichtlich aus den folgenden Tab. II und III).

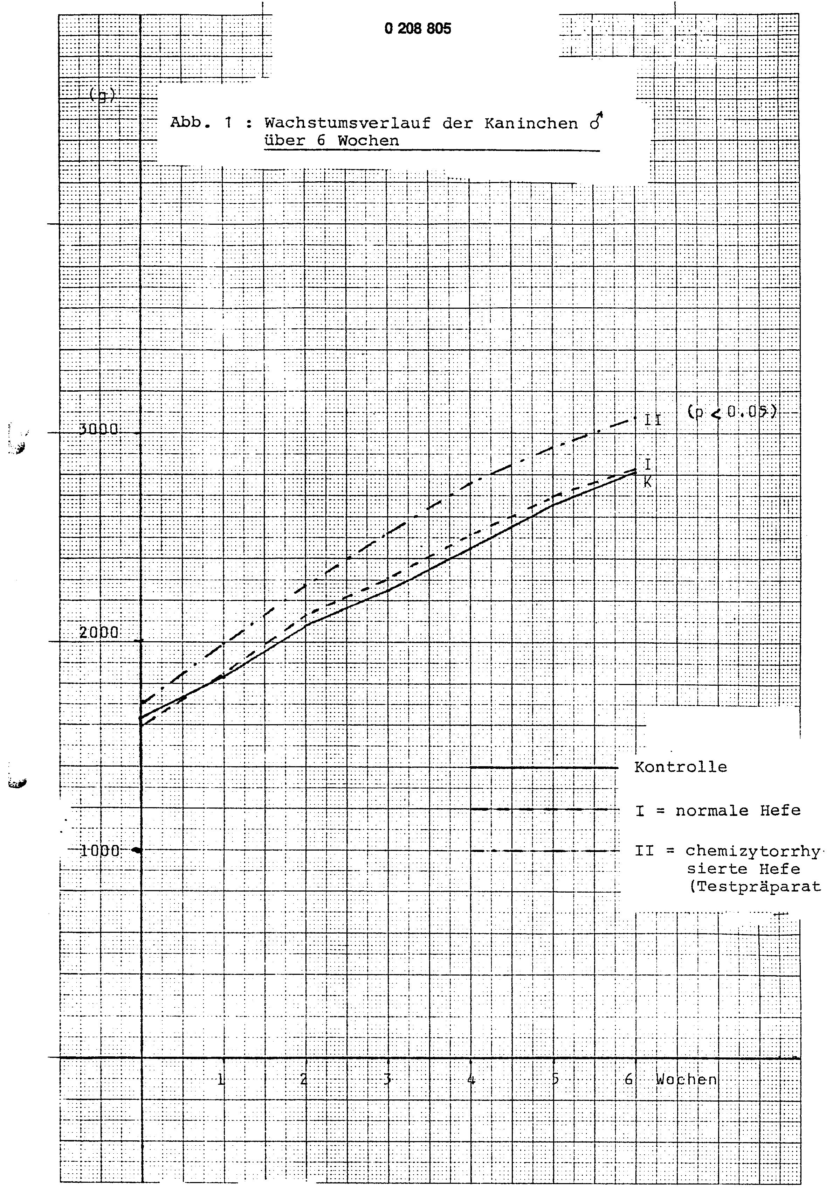

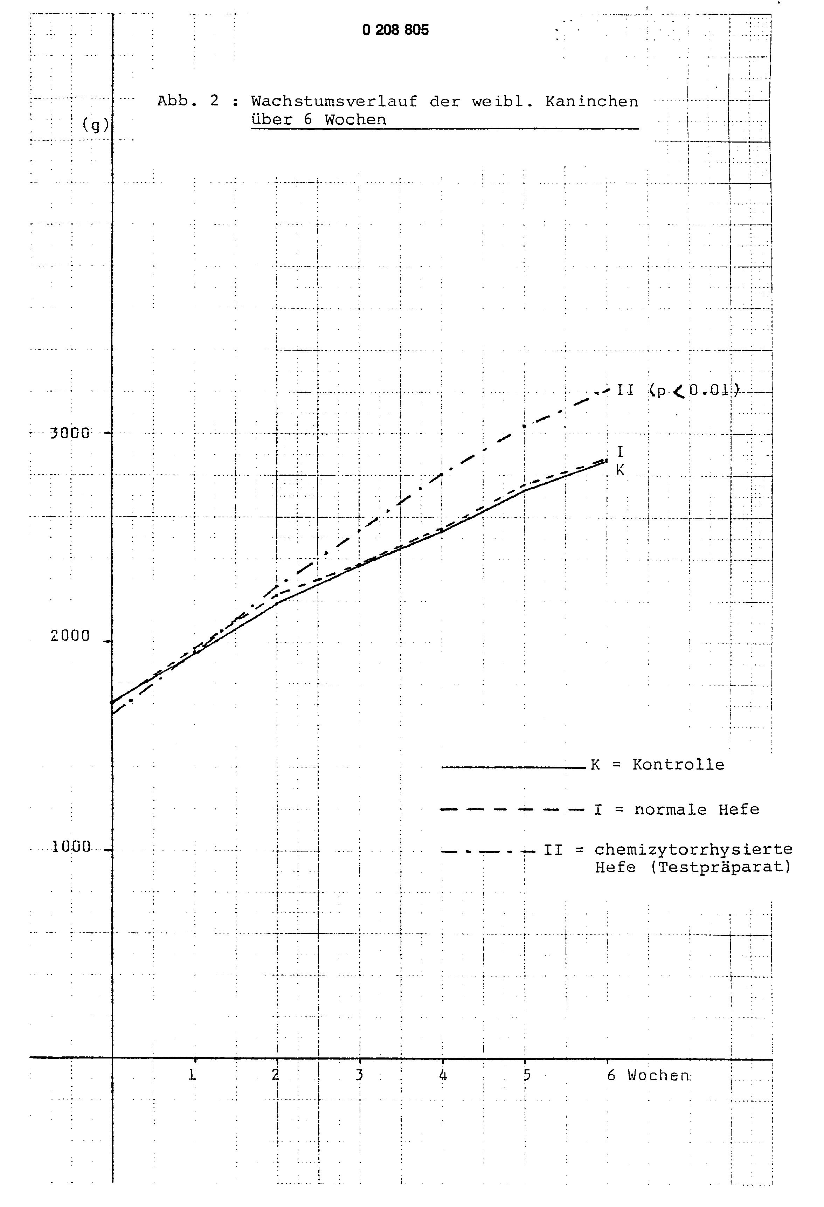

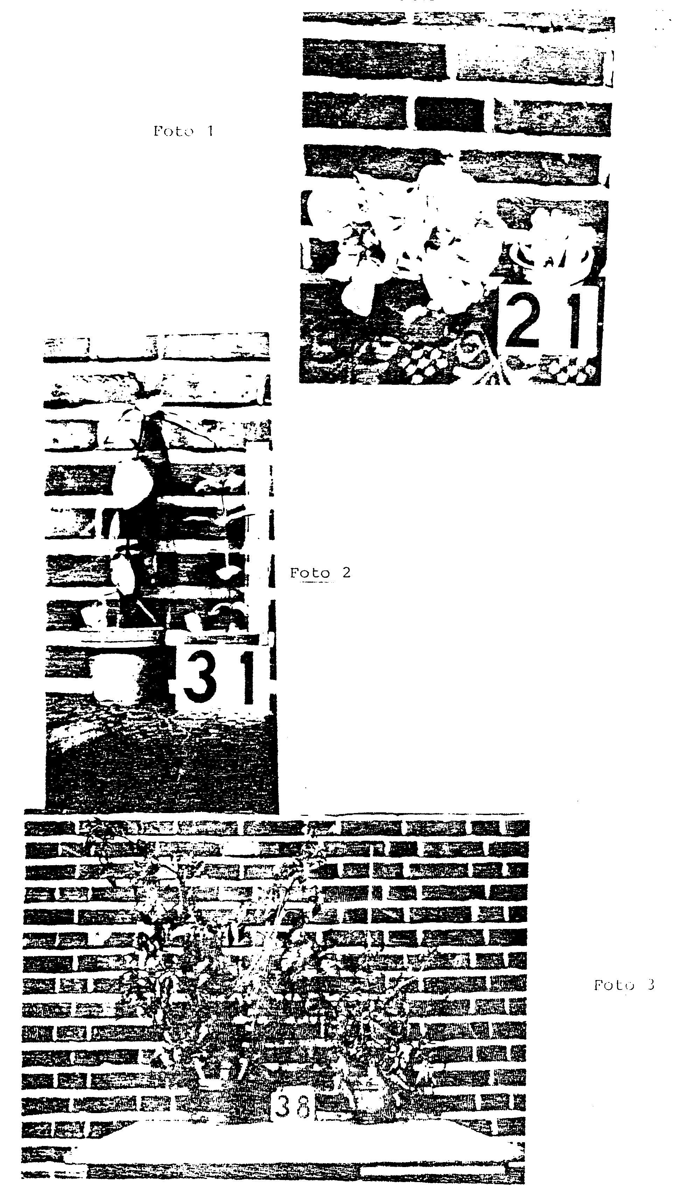

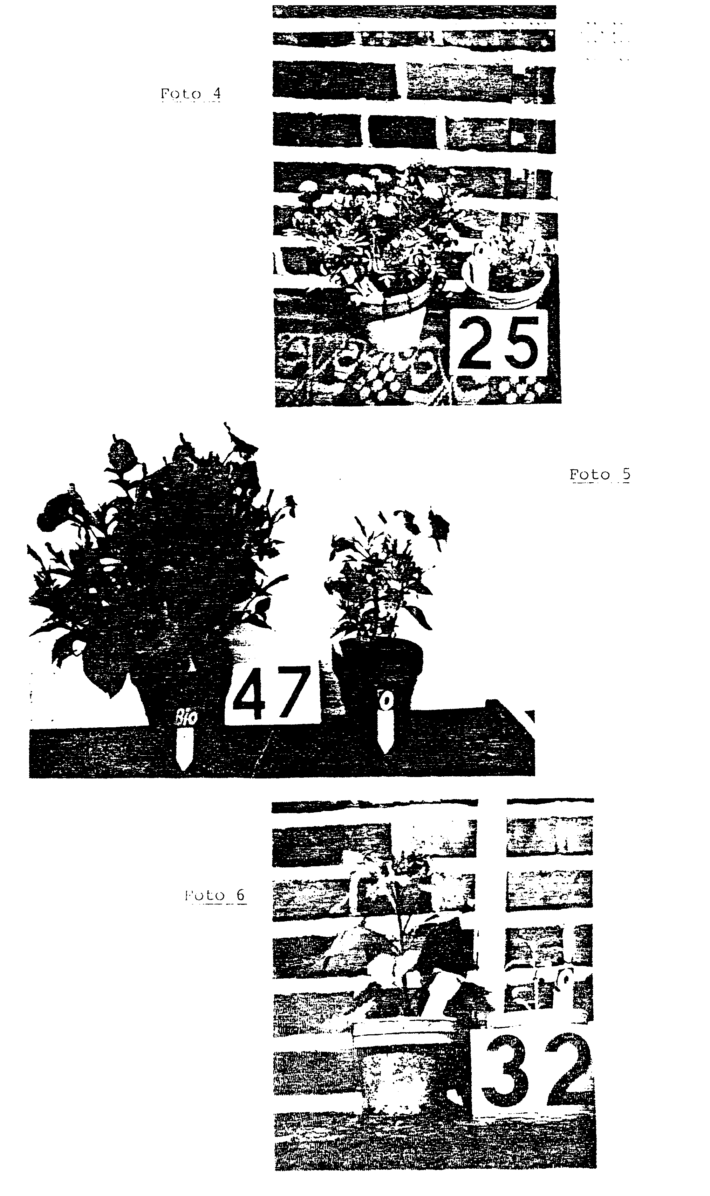

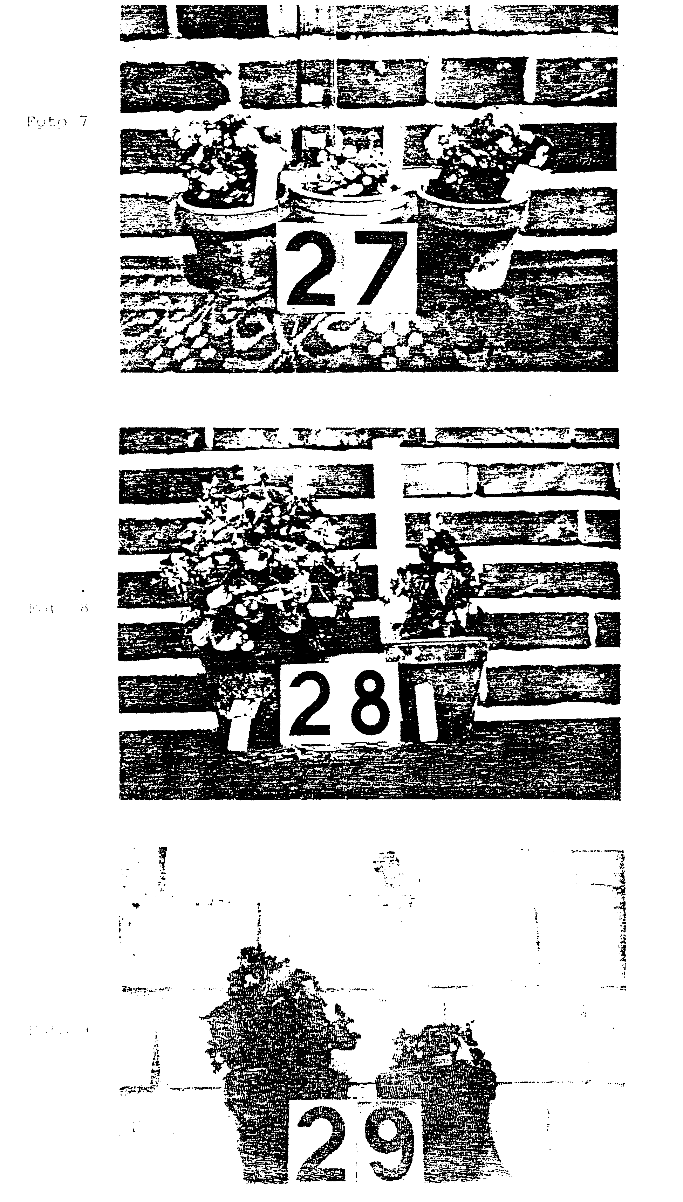

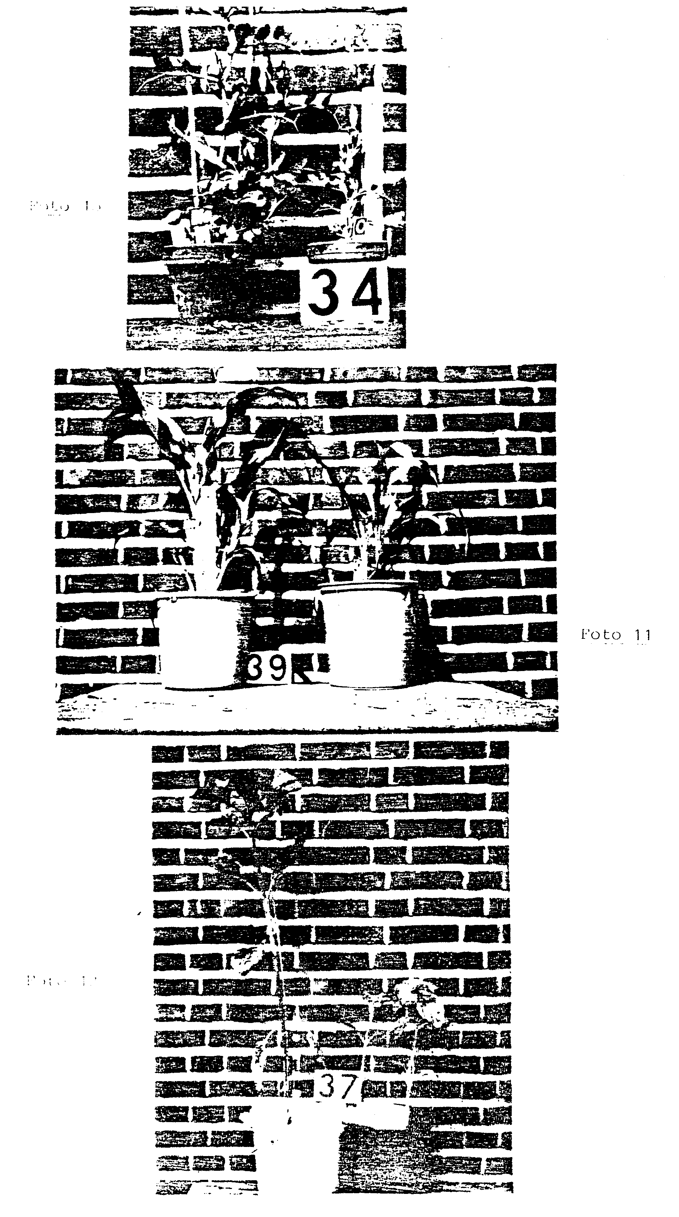

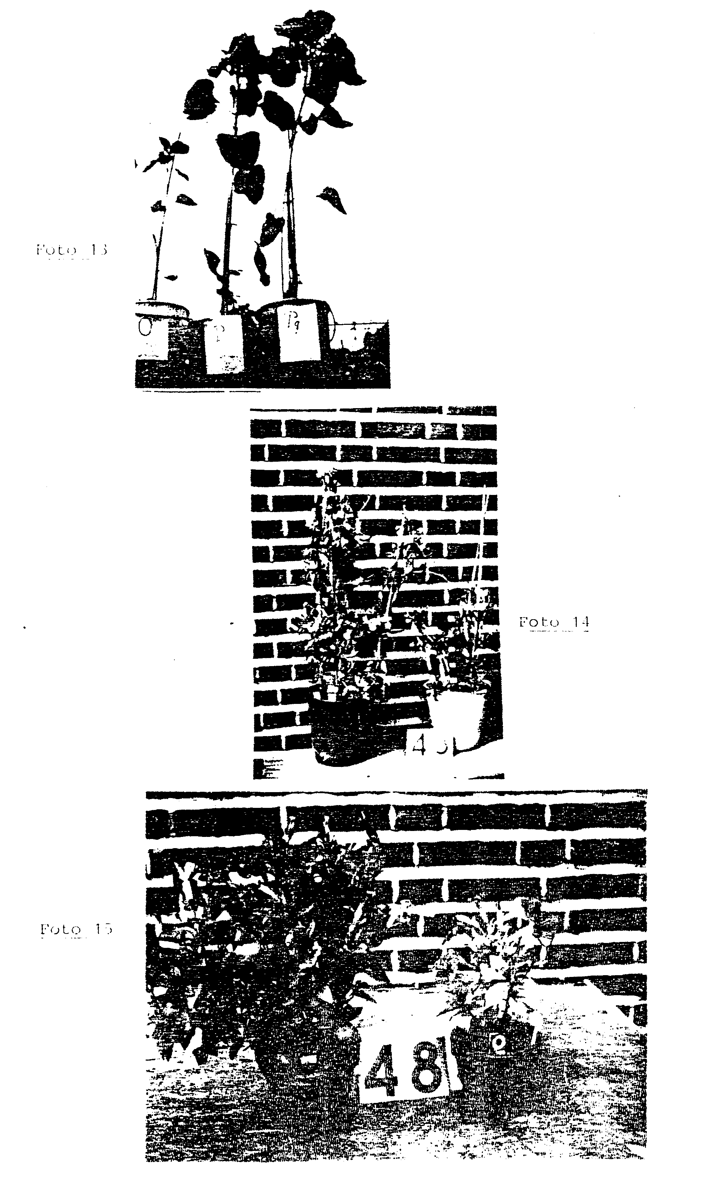

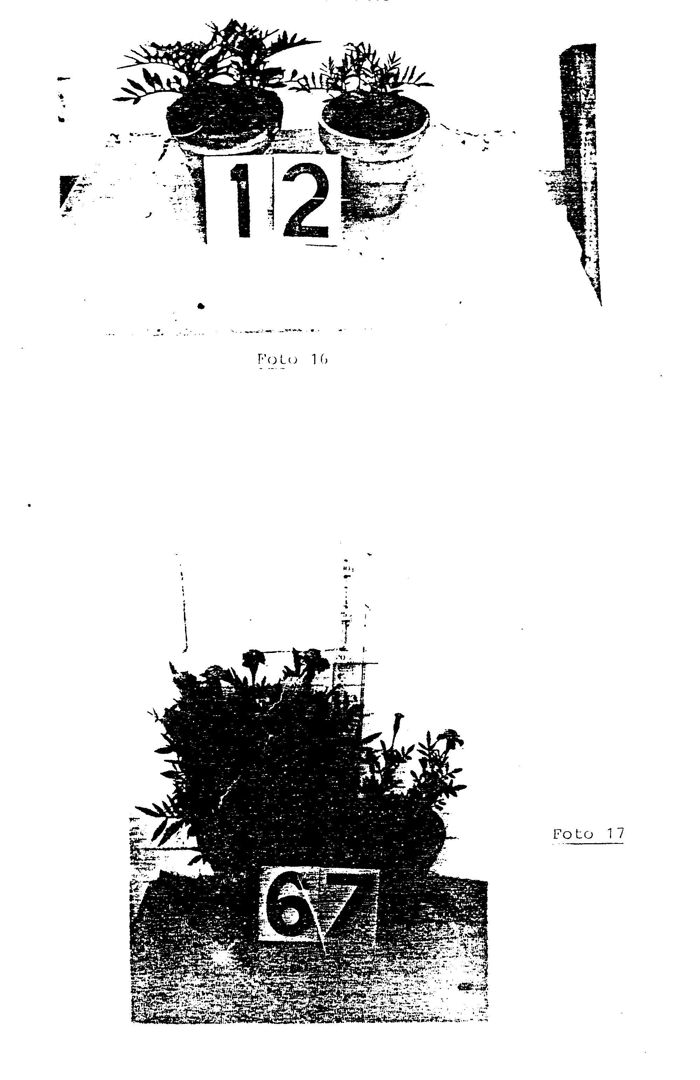

Körpergewichte: Männchen und Weibchen zeigten eine deutlich verbesserte Gewichtszunahme, die statistisch signifikant war (p kleiner als 0,05 für Männchen, kleiner als 0,01 für Weibchen), wie aus den Tab. IV und V ersichtlich ist. Die Resultate der Tab. IV und V sind in den entsprechenden Abb. 1 und 2 graphisch dargestellt.

Futterverbrauch/Wasserverbrauch: Keine wesentlichen Unterschiede im Totalverbrauch.

Sektionsbefunde: Keine Abweichungen von den Kontrolltieren (Tab. VI und VII).

Organgewichte: Bei der Endsekretion wurden die Organe Herz, Leber, beide Nieren und Nebennieren präpariert und gewogen. Für die statistische Auswertung werden die absoluten Organgewichte in Relation zu den Körpergewichten in relative Organgewichte transformiert.

Der statistische Vergleich der Gruppenmittel zeigte weder bei den Männchen noch bei den Weibchen statistische Unterschiede oder dosisabhängige Gruppentrends.

Eine Belastung der Hauptausscheidungsorgane durch das Testpräparat konnte damit ausgeschlossen werden, wie auch die Tab. VIII und IX belegen.

Ein Zusatz von 0,1 % zum Trinkwasser verursachte eine deutlich erhöhte Gewichtszunahme und damit eine verbesserte Futterausnutzung gegenüber den Kontrollen.

Tabelle II

Wochenbericht für klinische Beobachtungen

Testwoche: 1 - 6          Gruppe: Kontrolle          Tier-Nr.: 1 - 10     Sex: 5 ♂ 5♀

| Untersuchung makroskopisch | Befundzusammenstellung |
|---|---|
| Aussehen/Verhalten | keine Abweichungen von Normalbeobachtungen |
| Reflexe | keine Abweichungen von Normalbeobachtungen |
| Haarkleid/Hautturgor | keine Abweichungen von Normalbeobachtungen |
| Körperöffnungen | keine Abweichungen von Normalbeobachtungen |
| Faeces (Farbe, Konsistenz) | keine Abweichungen von Normalbeobachtungen |
| Körpergewichte Futter-/ Wasseraufnahme | keine Abweichungen von Normalbeobachtungen |
| Akute Symptome | keine Abweichungen von Normalbeobachtungen |

Tabelle III

Wochenbericht für klinische Beobachtungen

Testwoche: 1 - 6          Gruppe: II          Tier-Nr.: 1 - 10          Sex: 5 ♂ 5 ♀

| Untersuchung makroskopisch | Befundzusammenstellung |
|---|---|
| Aussehen/Verhalten | keine Abweichungen von Normalbeobachtungen |
| Reflexe | keine Abweichungen von Normalbeobachtungen |
| Haarkleid/Hautturgor | keine Abweichungen von Normalbeobachtungen |
| Körperöffnungen | keine Abweichungen von Normalbeobachtungen |
| Faeces (Farbe, Konsistenz) | keine Abweichungen von Normalbeobachtungen |
| Körpergewichte Futter-/ Wasseraufnahme | keine Abweichungen von Normalbeobachtungen |
| Akute Symptome | keine Abweichungen von Normalbeobachtungen |

0 208 805

Tabelle IV

Zusammenfassung: Körpergewichte (g) ♂

| Gruppe | | Anfangs-gewicht | 1. Woche | 2. Woche | 3. Woche | 4. Woche | 5. Woche | 6. Woche | Zunahme (Gesamt) |
|---|---|---|---|---|---|---|---|---|---|
| K | $\bar{x}$ | 1635 | 1829 | 2081 | 2247 | 2451 | 2652 | 2813 | 1174,9 |
| | $s \pm$ | 291 | 258 | 205 | 247 | 271 | 235 | 241 | 213,2 |
| II*) | $\bar{x}$ | 1712 | 1978 | 2278 | 2519 | 2762 | 2933 | 3077 | 1365,0**) |
| | $s \pm$ | 145 | 183 | 218 | 212 | 234 | 255 | 199 | 106,6 |

*) Testpräparat aus Beispiel 3

**)   $p < 0,05$

0 208 805

Tabelle V

Zusammenfassung: Körpergewichte (g) ♀

| Gruppe | | Anfangs-gewicht | 1. Woche | 2. Woche | 3. Woche | 4. Woche | 5. Woche | 6. Woche | Zunahme (Gesamt) |
|--------|---|-----------------|----------|----------|----------|----------|----------|----------|------------------|
| K | $\bar{x}$ | 1709 | 1943 | 2194 | 2365 | 2533 | 2727 | 2868 | 1160,1 |
| | s ± | 272 | 243 | 259 | 267 | 267 | 250 | 280 | 62,0 |
| II | $\bar{x}$ | 1655 | 1946 | 2274 | 2541 | 2809 | 3038 | 3213 | 1556,9* |
| | s ± | 124 | 100 | 125 | 134 | 148 | 135 | 139 | 58,3 |

* $p < 0,01$

II = Testpräparat aus Beispiel 3

0 208 805

Tabelle VI

Endsektion (makroskopische Befunde)

Gruppe = Kontrolle

| Organ | Tier-Nr. | Befunde |
|-------|----------|---------|
| ZNS | 1 - 10 (5 ♂ 5 ♀) | makroskopisch ohne versuchssp. Veränderungen |
| Lunge | " | makroskopisch ohne versuchssp. Veränderungen |
| Herz/Herzbeutel | " | makroskopisch ohne versuchssp. Veränderungen |
| Magen | " | makroskopisch ohne versuchssp. Veränderungen |
| Dünn-/Dickdarm | " | markoskopisch ohne versuchssp. Veränderungen |
| Leber | " | makroskopisch ohne versuchssp. Veränderungen |
| Milz | " | makroskopisch ohne versuchssp. Veränderungen |
| Nieren | " | makroskopisch ohne versuchssp. Veränderungen |
| Serosen/Gefäße | " | makroskopisch ohne versuchssp. Veränderungen |
| Lymphknoten | " | makroskopisch ohne versuchssp. Veränderungen |
| Keimdrüsen | " | makroskopisch ohne versuchssp. Veränderungen |

0 208 805

## Tabelle VII

Endsektion (makroskopische Befunde)

Gruppe II = Testgruppe

| Organ | Tier-Nr. | Befunde |
|---|---|---|
| ZNS | 1 - 10 ( 5 ♂ 5 ♀) | makroskopisch ohne versuchssp. Veränderungen |
| Lunge | " | makroskopisch ohne versuchssp. Veränderungen |
| Herz/Herzbeutel | " | makroskopisch ohne versuchssp. Veränderungen |
| Magen | " | makroskopisch ohne versuchssp. Veränderungen |
| Dünn-/Dickdarm | " | makroskopisch ohne versuchssp. Veränderungen |
| Leber | " | makroskopisch ohne versuchssp. Veränderungen |
| Milz | " | makroskopisch ohne versuchssp. Veränderungen |
| Nieren | " | makroskopisch ohne versuchssp. Veränderungen |
| Serosen/Gefäße | " | makroskopisch ohne versuchssp. Veränderungen |
| Lymphknoten | " | makroskopisch ohne versuchssp. Veränderungen |
| Keimdrüsen | " | makroskopisch ohne versuchssp. Veränderungen |

Tabelle VIII

Relative Organgewichte (%)

$\eth$

| | | Herz | Leber | Niere li. | Niere re. | Nebenniere li. | Nebenniere re. |
|---|---|---|---|---|---|---|---|
| K-Kontrolle | 1 | 0,23 | 3,92 | 0,28 | 0,32 | 0,006 | 0,005 |
| | 2 | 0,22 | 3,10 | 0,32 | 0,32 | 0,004 | 0,005 |
| | 3 | 0,25 | 3,97 | 0,28 | 0,28 | 0,006 | 0,004 |
| | 4 | 0,22 | 3,47 | 0,29 | 0,30 | 0,004 | 0,006 |
| | 5 | 0,23 | 3,50 | 0,31 | 0,29 | 0,004 | 0,005 |
| | $\bar{x}$ | 0,23 | $\bar{x}$ 3,59 | $\bar{x}$ 0,30 | $\bar{x}$ 0,30 | $\bar{x}$ 0,005 | $\bar{x}$ 0,005 |
| | s | 0,01 | s 0,36 | s 0,02 | s 0,02 | s 0,001 | s 0,001 |
| II*) | 1 | 0,22 | 3,95 | 0,29 | 0,32 | 0,004 | 0,006 |
| | 2 | 0,22 | 3,62 | 0,31 | 0,30 | 0,005 | 0,004 |
| | 3 | 0,22 | 3,09 | 0,32 | 0,30 | 0,004 | 0,003 |
| | 4 | 0,24 | 3,41 | 0,30 | 0,32 | 0,004 | 0,004 |
| | 5 | 0,25 | 3,98 | 0,28 | 0,29 | 0,004 | 0,003 |
| | $\bar{x}$ | 0,23 | $\bar{x}$ 3,61 | $\bar{x}$ 0,30 | $\bar{x}$ 0,30 | $\bar{x}$ 0,0042 | $\bar{x}$ 0,0040 |
| | s | 0,01 | s 0,38 | s 0,02 | s 0,01 | s 0,0004 | s 0,0010 |

*) II = Testpräparat

0 208 805

Ein entsprechender Versuch mit Meerschweinchen ergab eine Steigerung der Futtereffektivität um ca. 50 %.

Tabelle IX

Relative Organgewichte (%)

| ♀ | Herz | Leber | Niere li. | Niere re. | Nebenniere li. | Nebenniere re. |
|---|---|---|---|---|---|---|
| K-Kontrolle 1 | 0,25 | 3,72 | 0,30 | 0,30 | 0,004 | 0,005 |
| 2 | 0,26 | 3,24 | 0,31 | 0,32 | 0,005 | 0,005 |
| 3 | 0,20 | 3,84 | 0,33 | 0,32 | 0,005 | 0,004 |
| 4 | 0,19 | 3,21 | 0,28 | 0,30 | 0,004 | 0,004 |
| 5 | 0,23 | 3,03 | 0,33 | 0,30 | 0,005 | 0,004 |
| x̄ | 0,22 | 3,41 | 0,31 | 0,31 | 0,0050 | 0,0050 |
| s | 0,03 | 0,35 | 0,02 | 0,01 | 0,0010 | 0,0010 |
| II-*) | | | | | | |
| 1 | 0,22 | 3,06 | 0,29 | 0,26 | 0,004 | 0,004 |
| 2 | 0,22 | 3,84 | 0,26 | 0,28 | 0,006 | 0,003 |
| 3 | 0,24 | 3,67 | 0,29 | 0,28 | 0,004 | 0,003 |
| 4 | 0,20 | 3,17 | 0,31 | 0,30 | 0,005 | 0,005 |
| 5 | 0,24 | 3,36 | 0,31 | 0,28 | 0,005 | 0,004 |
| x̄ | 0,22 | 3,53 | 0,29 | 0,28 | 0,0050 | 0,0040 |
| s | 0,02 | 0,26 | 0,02 | 0,01 | 0,0010 | 0,0010 |

*) II = Testpräparat

Futterausnutzung der Gruppen:

|  | Männl. Meer-schweinchen | Weibl. Meer-schweinchen |
|---|---|---|
| Kontrolle | 0,063 | 0,080 |
| Testgruppe | 0,093 | 0,123 |

Dies entspricht einer Verbesserung der Gruppe "Testversuch" um 51 %.

Bezüglich der Futter-und Wasseraufnahme wurden zwischen den beiden Gruppen keine wesentlichen Unterschiede festgestellt. Die Körpergewichtsverbesserung gegenüber den Kontrollen ergibt sich aus den Tab. X bis XIII.

## Tabelle X

Körpergewichte (g/Woche)                                                   Gruppe: Kontrolle - ♂♂

| Tier Nr. | Anfangsgewichte | 1. Woche | 2. Woche | 3. Woche | 4. Woche | 5. Woche | Zunahme |
|---|---|---|---|---|---|---|---|
| 1 | 786 | 790 | 799 | 840 | 870 | 910 | 126 |
| 2 | 470 | 490 | 540 | 550 | 570 | 590 | 120 |
| 3 | 460 | 475 | 500 | 510 | 530 | 570 | 110 |
| 4 | 510 | 540 | 550 | 590 | 635 | 655 | 144 |
| 5 | 614 | 630 | 640 | 675 | 705 | 735 | 120 |
| 6 | 674 | 700 | 714 | 745 | 780 | 805 | 130 |
| 7 | 450 | 505 | 535 | 560 | 585 | 620 | 170 |
| 8 | 540 | 545 | 590 | 615 | 630 | 655 | 115 |
| 9 | 490 | 525 | 535 | 570 | 605 | 630 | 140 |
| 10 | 506 | 525 | 550 | 585 | 615 | 660 | 155 |
| $\bar{x}$ | 550,0 | 572,5 | 595,5 | 624,0 | 652,6 | 683,0 | 133,0 |
| SD ± | 109,2 | 102,2 | 95,3 | 101,2 | 103,9 | 105,1 | 19,18 |



Tabelle XI

## Körpergewichte (g/Woche)

Testgruppe: - ♂♂

| Tier Nr. | Anfangsgewicht | 1. Woche | 2. Woche | 3. Woche | 4. Woche | 5. Woche | Zunahme |
|---|---|---|---|---|---|---|---|
| 1 | 490 | 544 | 585 | 605 | 670 | 695 | 206 |
| 2 | 455 | 505 | 540 | 575 | 645 | 670 | 215 |
| 3 | 470 | 490 | 535 | 570 | 610 | 645 | 175 |
| 4 | 560 | 585 | 635 | 670 | 710 | 745 | 185 |
| 5 | 480 | 520 | 560 | 595 | 635 | 675 | 195 |
| 6 | 640 | 670 | 695 | 735 | 760 | 795 | 155 |
| 7 | 610 | 635 | 674 | 710 | 740 | 785 | 175 |
| 8 | 470 | 495 | 540 | 565 | 585 | 635 | 165 |
| 9 | 675 | 685 | 730 | 770 | 795 | 830 | 155 |
| 10 | 520 | 550 | 570 | 606 | 670 | 680 | 160 |
| $\bar{x}$ | 537,0 | 568,0 | 606,5 | 640,0 | 682,0 | 715,5 | 178,5*) |
| SD ± | 79,6 | 72,6 | 71,9 | 75,2 | 67,9 | 68,2 | 21,09 |

*) $< 0,01$

## Tabelle XII

Körpergewichte (g/Woche)                                                           Gruppe: Kontrolle - ♀♀

| Tier Nr. | Anfangsgewicht | 1. Woche | 2. Woche | 3. Woche | 4. Woche | 5. Woche | Zunahme |
|----------|----------------|----------|----------|----------|----------|----------|---------|
| 1  | 570 | 625 | 630 | 650 | 695 | 735 | 165 |
| 2  | 550 | 620 | 660 | 660 | 700 | 720 | 170 |
| 3  | 495 | 520 | 535 | 555 | 580 | 605 | 110 |
| 4  | 710 | 755 | 760 | 770 | 805 | 840 | 130 |
| 5  | 400 | 420 | 465 | 505 | 530 | 560 | 160 |
| 6  | 380 | 395 | 435 | 475 | 520 | 545 | 165 |
| 7  | 410 | 435 | 470 | 505 | 510 | 550 | 140 |
| 8  | 670 | 675 | 690 | 730 | 750 | 785 | 115 |
| 9  | 560 | 605 | 615 | 645 | 655 | 690 | 130 |
| 10 | 460 | 505 | 550 | 585 | 635 | 665 | 205 |
| $\bar{x}$ | 520,5 | 555,5 | 581,0 | 608,0 | 638,0 | 669,5 | 149,0 |
| SD ± | 125,5 | 119,2 | 107,4 | 99,6 | 101,7 | 103,1 | 29,23 |

## Tabelle XIII

Körpergewichte (g/Woche)                                                                 Testgruppe: - ♀♀

| Tier Nr. | Anfangsgewicht | 1. Woche | 2. Woche | 3. Woche | 4. Woche | 5. Woche | Zunahme |
|---|---|---|---|---|---|---|---|
| 1 | 580 | 656 | 669 | 736 | 806 | 829 | 251 |
| 2 | 520 | 590 | 640 | 675 | 715 | 765 | 246 |
| 3 | 570 | 630 | 660 | 690 | 730 | 770 | 199 |
| 4 | 655 | 705 | 735 | 760 | 780 | 805 | 150 |
| 5 | 470 | 525 | 570 | 630 | 680 | 715 | 245 |
| 6 | 370 | 431 | 490 | 550 | 600 | 645 | 275 |
| 7 | 430 | 470 | 496 | 545 | 585 | 635 | 206 |
| 8 | 480 | 535 | 575 | 640 | 685 | 730 | 250 |
| 9 | 460 | 495 | 550 | 585 | 645 | 680 | 220 |
| 10 | 540 | 565 | 590 | 630 | 655 | 685 | 146 |
| $\bar{x}$ | 507,5 | 560,0 | 597,5 | 644,0 | 688,0 | 726,0 | 218,5*) |
| SD $\pm$ | 82,6 | 86,2 | 78,5 | 72,5 | 71,5 | 66,1 | 43,7 |

*) ⁓ 0,01

In den folgenden Beispielen werden bevorzugte Chemizytorrhysate hergestellt und als Nahrungszusatzstoffe bzw. Wuchsstoffe getestet. Einzelheiten zu den Abbildungen werden im Beispiel 4 erläutert.

Beispiel 1

5 kg Aspergillus niger und 5 kg Torula utilis werden bei 10°C unter Rühren in einer geeigneten Mischvorrichtung mit einer solchen Menge eines Salzgemisches versetzt, daß sich die Masse verflüssigt. Das Salzgemisch enthält Citrate und/oder Lactate und/oder Chloride der Alkalimetalle (Na, K) und Erdalkalimetalle (Mg, Ca) in einem Verhältnis von organischem Material zu Salz von 10 Gewichtsteilen zu 0,4 Gewichtsteilen, und zusätzlich 20 % Saccharose/Mannit (1:1), bezogen auf Salzmenge.

Durch Wärmezufuhr wird die Temperatur des Gemisches bis auf 56 -59°C gesteigert, um die Chemizytorrhyse abzuschließen. Zur Vermeidung von Kontaminationen wird die Suspension mit geeigneten Konservierungsstoffen, z.B. mit Nipagin (4-Hydroxybenzoesäuremethylester oder 4-Hydroxybenzoesäureethylester) versetzt. Das erhaltene Präparat enthielt ca. 35 % Trockensubstanz.

Der Wachstumstest an Guppifischen, die 1 Woche alt waren, ergab, wenn 2 o/oo des Präparates pro Woche dem normalen Futter hinzugefügt werden, daß die Tiere nach 4 Wochen 95 % größer als normal gefütterte Kontrollfische waren.

Beispiel 2

1 kg Neurospora crassa, 1 kg Lactobacillus bulgaricus und 10 kg japanische Ikashiokara-Hefe werden bei 30°C in einer Mischvorrichtung, die eine Einrichtung zum Erwärmen hat, mit 250 g einer Mischung aus Natriumphosphat ($Na_3PO_4$), Magnesiumphosphat, Natriumlactat, Glukose und Manganchlorid (Gewichtsverhältnis 1:0,1:0,05:0,05:0,001) und 125 g Saccharose + Mannit (1:1) versetzt. Unter Rühren tritt rasch Chemizytorrhyse ein, die durch Erwärmen auf eine Temperatur von 60°C beendet wird. Nach 1 Std. wird auf 20°C abgekühlt mit 100 g Kieselgel Aerosil 300 (feinst gemahlen, Staub,mittl. Durchmesser der Primärteilchen 7nm) und mit Nipagin/Hydroxychinolin (zwecks Konservierung) versetzt.

Das so hergestellte Präparat wird dem Futter von Pulcini White mountain Chicken in einer Menge von 0,1 % (Gewichtsbasis) hinzugefügt. Kontrolltiere und Versuchstiere werden 75 Tage lang gefüttert und am Tag 40 und Tag 75 gewogen:

| Gruppe | nach 40 Tagen Tierzahl | Gewicht (kg) | nach 75 Tagen Tierzahl | Gewicht (kg) |
|---|---|---|---|---|
| Kontrollgruppe | 965 | 1.180 | 970 | 1.991 |
| Versuchsgruppe | 990 | 1.258 | 989 | 2.190 |

Der Futterverbrauch pro kg Körpergewicht nach 75 Tagen ergibt sich wie folgt:

Kontrollgruppe 2,73 kg

Versuchsgruppe 2,48 kg

entsprechend einer Futterersparnis von ca. 10 %. Die Mortalitäten in der Versuchsgruppe sind geringer als in der Kontrollgruppe, d.h. die Gesundheit der Chicken wird günstig beeinflußt.

Auch der Protein-und Fettgehalt und die Trypsinverdauung des Fleisches der Versuchstiere sind wesentlich besser als die der Kontrollgruppe:

|  | % Protein | % Fett | % Verdaulichkeit durch Trypsin |
|---|---|---|---|
| Kontrollgruppe | 22,3 | 0,79 | 1,98 |
| Versuchsgruppe | 24,2 | 0,48 | 1,50 |

Nur 1,5 % des Fleisches bleiben bei 90stündiger Trypsinbehandlung des Fleisches der Versuchstiere unverdaut (gegenüber 1,98 % bei den Kontrolltieren)

Beispiel 3

5 kg Saccharomyces cerevisiae werden bei 35°C mit einem Gemisch aus 100 g NaCl, 15 g MgSO₄ und 25 g Rohrzucker (Saccharose) versetzt und bis auf 60°C erwärmt.

Ein geeignetes Antischaummittel wird hinzugefügt.

Nach einer ausreichenden Erhitzungszeit wird abgekühlt und mit Nipagin/Benzylakohol und/oder mit Germall konserviert.

Um die Suspension in eine feste Form zu bringen, wird sie mit der ausreichenden Menge feinteiligem Kieselgelstaub (vgl. Beispiel 2) aufgenommen. Ein zweckmäßiges Mengenverhältnis ist 2 ml Hefesuspension pro 1-3 g Aerosil 3oo.

Dem Futter von Ratten wurden ca. o,2 bis o,5 % des so erhaltenen Feststoffpräparats hinzugefügt. Es ergab sich eine verminderte Futteraufnahme und damit eine verbesserte Futterausnutzung.

Die Wachstumsparameter waren: Körpergewichte, Futterverbrauch, Wasseraufnahme, Organgewichte. Nahrung: Labor-Standarddiät Altromin.

Bedingungen: Raumtemperatur 22°C, rel. Luftfeuchtigkeit 50 -60 %, tägl. Beleuchtungsdauer 12 Stdn.. Körpergewichte und Futterverbrauch sind in den Tab. XIV bis XVI zusammengefaßt.

Futtereffektivität: Die FE-Zahl ist bei Erhöhung ein Hinweis entweder auf erhöhte Gewichtszunahme oder verminderten Futterverbrauch.

Da der Futterverbrauch in der Testgruppe signifikant niedriger lag als die Gewichtszunahme der Kontrolle entsprach, kann hier von einer verbesserten Futterausnutzung ausgegangen werden.

Die Futtereffektivitäten ergaben sich wie folgt:

Kontrolle: 0,21

Testgruppe: 0,24

jeweils ausgedrückt als Gewichtszunahme in 6 Wochen pro 1g Futteraufnahme.

Tabelle XIV

Zusammenfassung: Körpergewichte (g)

| Gruppe | Anfangs-gewicht | 1. Woche | 2. Woche | 3. Woche | 4. Woche | 5. Woche | 6. Woche | Zunahme (Gesamt) |
|---|---|---|---|---|---|---|---|---|
| Kontrolle | 87,9 | 129,2 | 168,8 | 199,2 | 227,2 | 249,0 | 261,8 | 173,9 |
| | ± 3,7 | ± 5,6 | ± 7,3 | ± 9,0 | ± 13,2 | ± 18,1 | ± 22,1 | ± 21,1 |
| Testgruppe | 86,6 | 124,8 | 165,8 | 198,2 | 222,8 | 244,3 | 262,5 | 175,9 |
| | ± 3,6 | ± 4,9 | ± 5,5 | ± 6,3 | ± 8,4 | ± 9,8 | ± 10,2 | ± 11,9 |

0 208 805

Tabelle XV

Zusammenfassung: Futterverbrauch (g/Woche)

| Gruppe | 1. Woche | 2. Woche | 3. Woche | 4. Woche | 5. Woche | 6. Woche | Gesamt |
|--------|----------|----------|----------|----------|----------|----------|--------|
| Kontrolle | 112,5 | 128,7 | 147,5 | 135,0 | 141,2 | 152,0 | 816,8 |
| | ± 2,5 | ± 3,3 | ± 4,1 | ± 8,3 | ± 10,5 | ± 12,0 | ± 34,2 |
| Testgruppe | 103,3 | 115,3 | 132,8 | 126,2 | 134,8 | 146,8 | 770,9* |
| | ± 3,6 | ± 7,6 | ± 3,1 | ± 7,4 | ± 9.9 | ± 12,8 | ± 43,0 |

* p $< 0,05$

0 208 805

Tabelle XVI

Individueller Futterverbrauch über 6 Wochen

| Gruppe | Tier-Nr. | Gesamt (g) |
|---|---|---|
| Kontrolle | 1 | 818 |
| | 2 | 797 |
| | 3 | 772 |
| | 4 | 836 |
| | 5 | 872 |
| | 6 | 810 |
| Testgruppe | 1 | 725 |
| | 2 | 723 |
| | 3 | 793 |
| | 4 | 816 |
| | 5 | 816 |
| | 6 | 752 |

Beispiel 4

Es wurde wie in Beispiel 3 gearbeitet, jedoch wurde das Präparat nicht durch Aufnehmen in Kieselgel, sondern durch Sprühtrocknung verfestigt und in fester Form dem Futtermittel beigemengt.

Die Futterersparnis bei Hühnern, denen o,1 % festes Chemizytorrhysat zum Futter gegeben worden war, betrug 2o %. Der Kot der Hühner war fester, was für eine bessere Futterausnutzung sprach. Das Fleisch der Hühner hatte einen höheren Eiweißgehalt und einen geringeren Fettgehalt als das Fleisch der Hühner der Kontrollgruppe.

Beispiel 5

In diesem Beispiel wurde ein erfindungsgemäß hergestelltes Chemizytorrhysat verwendet, das wie folgt erhalten wurde:

100 Gewichtsteile eines Gemisches, das Weinhefe, Bäckerhefe und untergärige Bierhefe im Verhältnis von 8:90:2 enthält, wird unter Rühren mit 2,5 Gewichtsteilen eines Gemisches versetzt, das die Chloride des Kaliums, Magnesiums und Natriums sowie Saccharose und Glukonsäure im Verhältnis von 0,3:0,2:1,7:0,2:0,1 enthält.

Anfangstemperatur: 22°C. Endtemperatur nach raschem Erwärmen bis auf 52-62°C. Bei 40°C werden 2 Gewichtsteile feinstgemahlenes Kieselgel - (vgl. Beispiel 2) und 5 Gewichtsteile Mannit zugefügt. Nach Beendigung der Schaumbildung und nach Abkühlen auf 20°C ist das Präparat unmittelbar verwendbar, wenn man es dem Gießwasser hinzugibt.

Wenn das Präparat längere Zeit aufbewahrt werden muß, ist es zweckmäßig, ein Konservierungsmittel in einer Menge von 0,1 bis 0,3 % zuzusetzen. Das Präparat ist dann mindestens 1 Jahr haltbar. Vor Gebrauch ist immer umzuschütteln, da es sich um eine Suspension handelt.

Zur Bewertung des Chemizytorrhysats als Pflanzenwuchsstoff wurde folgende Versuchanordnung gewählt: Als Pflanzenerde wurde 1 Teil feiner Sand, 1 Teil Torf verwendet. Gepflanzt wurde am 20.5., soweit nichts anderes vermerkt. Das Präparat (Chemizytorrhysat) wurde dem Gießwasser in einer Menge von 1 Promille zugesetzt.

Die anliegenden Photos zeigen in Gegenüberstellung eine mit dem Chemizytorrhysat der Erfindung behandelte Pflanze (jeweils links) im Vergleich zu einer Kontroll-pflanze (jeweils rechts). Im einzelnen sind abgebildet:

Foto 1: Kopfsalat (Setzling am 20.5.)
Foto 2: Zinnia (Setzling am 20.5.)

Foto 3: Tomaten (Setzling am 20.5. 20 cm groß)

Foto 4: Tagetes (Setzlinge am 20.5. 15 -20 cm groß)

Foto 5: Petunien (Setzlinge am 20.5. 20 cm groß)

Foto 6: Sommeraster (Setzling am 20.5. 3 cm groß)

Foto 7: Ageratum (Setzlinge am 20.5. 5 cm groß) links und rechts erfindungsgemäßes Präparat, Mitte = Kontrolle

Foto 8: Begonia (Setzlinge am 20.5. 15 cm groß)

Foto 9: Begonia (Setzlinge am 20.5. 15 cm groß)

Foto 10: Erbsen (aus Samen gepflanzt am 25.4./5.5.)

Foto 11: Mais (aus Samen gepflanzt am 25.4./5.5.)

Foto 12: Sonnenblumen (aus Samen gepflanzt am 25.4./5.5.)

Foto 13: Sonnenblumen (aus Samen gepflanzt am 25.4.)

Foto 14: Erbsen (aus Samen gepflanzt am 25.4.)

Foto 15: Petunien (Setzlinge am 25.4. 15 cm groß)

Foto 16: Tagetes erecta (Setzlinge im Mai gepflanzt)

Foto 17: Tagetes erecta (Setzlinge im Mai gepflanzt)

Die Abbildungen sind Fotografien, aufgenommen am 1.7., d.h. nach ca. 6 Wochen Gießen mit der Präparatelösung, sofern die Setzlinge am 20.5. gepflanzt wurden. Foto 16 wurde am 16.6. und Foto 17. am 8.7. aufgenommen.

Man erkennt, daß das erfindungsgemäß hergestellte Chemizytorrhysat zu einem wesentlichen größeren Wuchs der jeweiligen Pflanzen führt.

### Beispiel 6

1 kg Chlorella, 1o kg Candida utilis, 1 kg Sporobolomycetes (sämtlich frisch und feucht), 1 kg Phytophora fungi und 1 kg Spirillum werden wie in Beispiel 3 beschrieben behandelt, indem 29o g NaCl, 6o g $MgSO_4$ . $7H_2O$ und 3o g Disaccharid sowie 75 g Sorbit und o,1 g $MnSO_4$ addiert werden und im Verlauf von 15 Stunden auf 75°C erwärmt wird. Nach dem Abkühlen wird mit Benzylalkohol konserviert und das Produkt sprühgetrocknet.

1,4 Promille dieses Produktes werden in ein spezielles Lachsfutter eingearbeitet. Junge Lachse, die mit diesem Futter behandelt werden, zeigen gegenüber einer Kontrollgruppe (ohne diesen 1,4 ‰ -Zusatz) ein wesentlich schnelleres Wachstum. Der Futterverbrauch läßt sich hierdurch um ca. 3o % senken.

### Beispiel 7

Ein Gemisch aus Hansenula anomala Hansen-Sydow (feucht, frisch), Trichosporum cutaneum OTA und feuchter frischer S. fragilis (Verhältnis 1 : 1 : 15) wird mit 2,5 % eines Gemisches aus KCl, $MgSO_4$ . $7H_2O$, NaCl, Sorbit, Glucose, Rohrzucker, $CoCl_2$ (Verhältnis entsprechend o,oo5 : o,1 : 1,o : o,o1 : o,o1 : o,o3 : : o,oo oo4) innig vermischt und unter Rühren im Verlauf von 3 bis 9 Stunden auf 50°C und nach und nach auf 62°C erhitzt.

Nach Zusatz von Kieselgel und Konservierungsmitteln wird sprühgetrocknet, wobei eine Temperatur unter 9o°C einzuhalten ist. Das Produkt eignet sich als Futtermittel-Zusatzstoff zur Aufzucht von Speisefischen aller Art, aber auch zur Aufzucht von Ferkeln, Gänsen, Hühnern und Kälbern. Im Falle der Anwendung in Milch (Kälber) kann das Produkt zu 2 Promille in der als Suspension anfallenden Form direkt zum Einsatz gebracht werden, d.h. ohne Sprühtrocknung.

Da sprühgetrocknetes Produkt ebenso wie die Suspensionsform die Darmflora von Tieren reguliert, eignet sich das Präparat hervorragend zur Behandlung von Diarrhoe, z.B. bei transportgeschädigten Rindern usw.. Wenige Tage der Behandlung reichen aus, um wieder eine Normalisierung zu erreichen.

### Ansprüche

1. Verfahren zur Herstellung eines thallophytischen oder bryophytischen Zytorrhysats, dadurch gekennzeichnet, daß intakte Thallophyt-und/oder Bryophytzellen chemizytorrhytisch behandelt werden, indem die Zellen bei Temperaturen im Bereich von 0°C bis 40°C innig mit einem wasserlöslichen Salz oder Salzgemisch von Metallen der 1. und 2. Hauptgruppe des Periodensystems in einer zur Verflüssigung der Zellkörpermasse ausreichenden Menge gemischt werden und das verflüssigte Gemisch anschließend auf eine Temperatur erwärmt wird, die über 40°C, jedoch unterhalb der Pasteurisierungstemperatur liegt, und dann abgekühlt und gegebenenfalls verfestigt wird.

2. Verfahren nach Anspruch 1, dadurch gekennzeichnet, daß das verflüssigte Gemisch auf eine Temperatur im Bereich von 40°C bis 70° erwärmt wird.

3. Verfahren nach Anspruch 1 oder 2, dadurch gekennzeichnet, daß auf eine Temperatur von höchstens 63°C erwärmt wird.

4. Verfahren nach einem der Ansprüche 1 bis 3, dadurch gekennzeichnet, daß das verflüssigte Gemisch gleichmäßig und rasch erwärmt wird, bevorzugt mit einer Geschwindigkeit von mindestens 10°/min.

5. Verfahren nach einem der Ansprüche 1 bis 4, dadurch gekennzeichnet, daß das Gemisch mindestens 10 Minuten erwärmt wird.

6. Verfahren nach einem der Ansprüche 1 bis 5, dadurch gekennzeichnet, daß dem Gemisch zusätzlich Kohlenhydrate und/oder Alkohole zugesetzt werden.

7. Verfahren nach einem der Ansprüche 1 bis 6, dadurch gekennzeichnet, daß ein Salzgemisch verwendet wird, das Natriumsalz(e) und Calcium- und/oder Magnesiumsalz(e) enthält.

8. Verfahren nach Anspruch 7, dadurch gekennzeichnet, daß die Salze als Sulfat, Nitrat, Chlorid, Acetat, Citrat, Lactat, Malat, Succinat, Carbonat, Bicarbonat und/oder Phosphat verwendet werden.

9. Verfahren nach einem der Ansprüche 1 bis 8, dadurch gekennzeichnet, daß mit etwa 1,5 bis 2,5 Gewichtsteilen Salz, bezogen auf 100 Gewichtsteile intakte Zellkörpermasse, gemischt wird.

10. Verfahren nach einem der Ansprüche 1 bis 9, dadurch gekennzeichnet, daß dem Gemisch Glukose und/oder Saccharose in einer Menge von 0,5 bis 1,5 Gewichtsteilen pro 100 Gewichtsteile intakte Zellkörpermasse zugesetzt werden.

11. Verfahren nach einem der Ansprüche 1 bis 10, dadurch gekennzeichnet, daß das Zellmaterial aus der Gruppe: Aspergillus niger, Torula utilis, Neurospora crassa, Lactobacillus bulgaricus, Saccharomyces-Arten und japanische Ikashiokara-Hefe ausgewählt ist.

12. Verfahren nach einem der Ansprüche 1 bis 11, dadurch gekennzeichnet, daß als Zellmaterial Saccharomyces cerevisiae verwendet wird.

13. Verfahren nach einem der Ansprüche 1 bis 12, dadurch gekennzeichnet, daß dem Zellmaterial etwa 2 Gew.-% Alkalichlorid, bevorzugt NaCl, etwa 0,3 Gew.-% Erdalkalisalz, bevorzugt $MgSO_4 \cdot 7 H_2O$, und etwa 0,5 Gew.-% Disaccharid, bevorzugt Saccharose, zur Verflüssigung zugesetzt werden.

14. Verfahren nach einem der Ansprüche 1 bis 13, dadurch gekennzeichnet, daß das chemizytorrhysierte Gemisch in eine feste Form überführt wird.

15. Verfahren nach Anspruch 14, dadurch gekennzeichnet, daß das Gemisch durch milde Sprühtrocknung verfestigt wird.

16. Chemizytorrhysat, erhalten nach einem Verfahren der Ansprüche 1 bis 15.

17. Verwendung des Chemizytorrhysats nach Anspruch 16 zur Nährstoffergänzung zum Futter, zur Nahrung und/oder zum Trinkwasser, insbesondere in Mengen von etwa 0,1 bis 0,5% bezogen auf die Nährstoffgrundlage.

18. Verwendung des Chemizytorrhysats nach Anspruch 16 als Pflanzenwuchsstoff.

19. Verwendung des Chemizytorrhysats nach Anspruch 16 als Kosmetikwirkstoff zur Aktivierung des Hautstoffwechsels.

20. Verwendung des Chemizytorrhysats nach Anspruch 16 als Aphrodisiakum zur Steigerung der Potenz.

21. Verwendung des Chemizytorrhysats nach Anspruch 16 als Darmregulans für Säugetiere, einschließlich Menschen.

Abb. 1 : Wachstumsverlauf der Kaninchen ♂ über 6 Wochen

**(g)**

Abb. 2 : Wachstumsverlauf der weibl. Kaninchen
über 6 Wochen

II (p < 0.01)

I
K

3000

2000

—————————— K = Kontrolle

— — — — — I = normale Hefe

—·—·—·— II = chemizytorrhysierte
Hefe (Testpräparat)

1000

1        2        3        4        5        6 Wochen

Foto 1

21

Foto 2

31

Foto 3

38

Foto 4

Foto 5

Foto 6

Foto 7

Foto 8

Foto 10

Foto 11

Foto 12

Foto 13

Foto 14

Foto 15

Foto 16

Foto 17

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 062 866 (DE LAREBEYRETTE) <br> * Ansprüche 1,2,5 * | 1,7,8, 11,17 | C 12 N 5/00 <br> C 12 N 1/06 <br> A 01 N 63/00 <br> A 01 N 65/00 <br> A 23 K 1/16 <br> A 23 L 1/30 <br> A 61 K 7/48 <br> A 61 K 35/66 <br> A 61 K 35/72 <br> A 61 K 35/78 |
| A | DE-A-2 414 521 (HITACHI) <br><br> * Ansprüche 1,3,7 * | 1,11, 17 | |
| A | CHEMICAL ABSTRACTS, Band 84, Nr. 8, 23. Februar 1976, Seite 381, Spalte 1, Abstrakt Nr. 49646p, Columbus, Ohio, US; & JP - A - 75 - 124 464 (ASAHI CHEMICAL INDUSTRY) 30.09.1975 | 1,2 | |

RECHERCHIERTE
SACHGEBIETE (Int. Cl.4)

A 01 N 63/00
A 01 N 65/00
A 23 K 1/16
A 23 L 1/30
A 61 K 7/48
A 61 K 35/66
A 61 K 35/72
A 61 K 35/78
C 07 G 17/00
C 12 N 1/06
C 12 N 5/00

Der vorliegende Recherchenbericht wurde für alle Patentansprüche erstellt.

| Recherchenort | Abschlußdatum der Recherche | Prüfer |
|---|---|---|
| BERLIN | 10-03-1986 | KNAACK M |